# EUROPEAN PATENT SPECIFICATION

(11) **EP 0 529 342 B1**
(45) Date of publication and mention of the grant of the patent: **13.03.1996**
(21) Application number: 92113092.8
(22) Date of filing: 31.07.1992
(51) Int. Cl.: C07C 17/38, C07C 19/10, C01B 7/19

(54) **Removal of hydrogen fluoride from mixture of hydrogen fluoride and dichlorofluoromethane**
Entfernung von Fluorwasserstoff aus einem Gemisch von Fluorwasserstoff und Dichlorfluormethan
Elimination de fluorure d'hydrogène d'un mélange de fluorure d'hydrogène avec dichlorofluorométhane

(30) Priority: 31.07.1991 JP 191762/91
(43) Date of publication of application: 03.03.1993
(73) Proprietor: DAIKIN INDUSTRIES, LTD., Kita-ku, Osaka 530 (JP)
(72) Inventor: Tsuda, Takehide, c/o Yodogawa Works of, Settsu-shi, Osaka-fu (JP); Komatsu, Satoshi, c/o Yodogawa Works of, Settsu-shi, Osaka-fu (JP); Koyama, Satoshi, c/o Yodogawa Works of, Settsu-shi, Osaka-fu (JP)
(74) Representative: Hansen, Bernd, Dr. Dipl.-Chem.

(56) References cited:
- EP-A- 98 341
- EP-A- 353 970
- EP-A- 354 697
- US-A- 2 450 414

## Description

The present invention relates to a process for removing hydrogen fluoride (hereinafter referred to as "HF") from a mixture of HF and dichlorofluoromethane (hereinafter referred to as "R-21") to obtain concentrated R-21.

### Description of the Related Art

Usually, R-21 is prepared by reacting chloroform and HF, and a resulting reaction mixture is washed with an aqueous liquid to remove HF from the reaction mixture. However, this washing method is not attractive, since a considerable amount of an alkali is required for neutralization of HF in the aqueous liquid.

US-A-2 450 414 refers to a process of separating valuable components from a reaction mixture comprising HCl, HF, CHClF₂ and CHCl₂F, which comprises the steps of subjecting the reaction mixture to anhydrous fractional distillation to distill off the HCl as substantially pure anhydrous hydrogen chloride, then subjecting the remaining mixture to a further anhydrous fractional distillation to distill off a mixture comprising substantially the monochlorodifluoromethane and part of the hydrogen fluoride, settling the undistilled mixture until it forms a layer of substantially anhydrous hydrogen fluoride containing a minor portion of dissolved dichloromonofluoromethane and a layer comprising dichloromonofluoromethane, and separating the layers.

### SUMMARY OF THE INVENTION

An object of the present invention is to provide a novel process for removing HF from a mixture comprising HF and R-21.

According to a first aspect of the present invention, there is provided a process for removing HF from a mixture comprising HF and R-21, which process comprises distilling said mixture and removing hydrogen fluoride in the form of an azeotropic mixture of hydrogen fluoride and dichlorofluoromethane.

According to a second aspect of the present invention, there is provided a process for removing HF from a mixture comprising HF and R-21, which process comprises cooling said mixture to a temperature of 70°C or lower to separate the mixture to form an upper liquid phase rich in hydrogen fluoride and a lower liquid phase rich in dichlorofluoromethane and distilling said lower liquid phase to remove hydrogen fluoride in the form of an azeotropic mixture of hydrogen fluoride and dichlorofluoromethane.

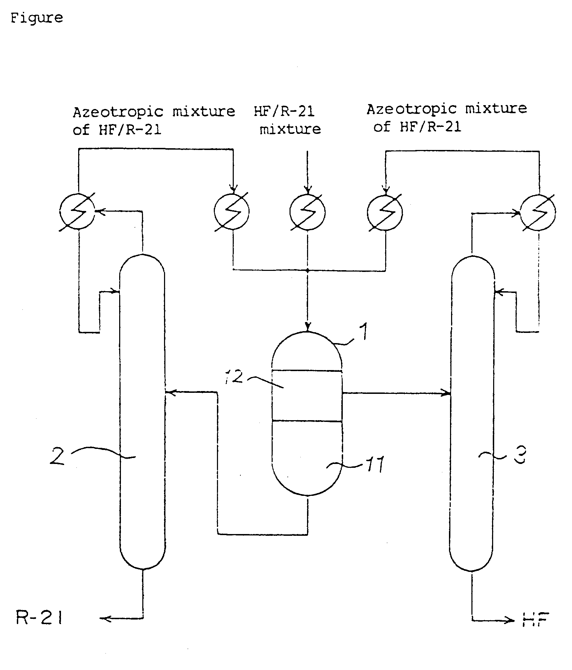

### BRIEF DESCRIPTION OF THE DRAWING

Figure schematically shows an example of an apparatus for carrying out the process of the present invention.

### DETAILED DESCRIPTION OF THE INVENTION

Since a mixture of HF and R-21 in a certain ratio forms an azeotropic mixture, R-21 cannot be concentrated to a molar ratio of 70/30 (HF/R-21) or higher. That is, a liquid phase in this molar ratio has the same composition as that of a liquid phase which is in an equilibrium state. When an attention is paid on the ratio of HF to R-21 during the phase separation of the mixture of HF and R-21 by cooling, a ratio of R-21 to HF in the lower liquid phase is larger than that in the mixture before phase separation. Thereby, the composition of the lower liquid phase shifts to a composition containing R-21 in a larger ratio than in the azeotropic mixture.

For phase separation, a cooling temperature is 70°C or lower. At a temperature higher than 70°C, no phase separation occurs at any ratio of HF to R-21. A preferred temperature range is 50°C or lower. A lower limit of the cooling temperature is a freezing point of R-21, namely about -135°C, preferably -50°C. A more preferred temperature range is between -30°C and 30°C, in particular between -10°C and 10°C.

In the present invention, HF can be removed from the mixture of HF and R-21 by directly distilling the mixture using a distillation apparatus. Any distillation apparatus can be used insofar as it has necessary functions for distillation. A simple distillation apparatus or a rectification apparatus having a plate column may be used and the latter is preferred. The distillation can be carried out batchwise or continuously. When the content of HF in the mixture is richer than that in the azeotropic composition, the azeotropic mixture of HF and R-21 is removed from the top of the distillation apparatus, and HF containing substantially no R-21 is recovered from the bottom of the apparatus. When the content of R-21 in the mixture is richer than that in the azeotropic mixture, the azeotropic mixture of HF and R-21 is removed from the top of the apparatus, and R-21 containing substantially no HF is recovered from the bottom of the apparatus.

The process of the present invention is particularly useful for removing HF from a reaction mixture containing HF and R-21 obtained by fluorinating chloroform with HF in a gas or liquid phase in the presence of a catalyst.

### PREFERRED EMBODIMENTS OF THE INVENTION

The present invention will be illustrated by making reference to the accompanying drawing.

The Figure schematically shows an example of a separation apparatus for carrying out the process of the present invention.

In the above fluorination process, a reaction mixture is obtained in a liquid phase and contains R-21, HF, hydrogen chloride and a small amount of other organic compounds. After removing hydrogen chloride, the mixture containing R-21 and HF is supplied in a separation tank 1 through a cooling device and phase separated. A lower liquid phase 11 rich in R-21 is introduced in a first distillation apparatus 2, and an azeotropic mixture of HF and R-21 is removed from the top of the apparatus 2. At the bottom of the distillation apparatus 2, R-21 containing substantially no HF remains and is recovered as a product.

The azeotropic mixture which is removed from the top of the top of the apparatus 2 is again cooled and recycled to the separation tank and subjected to the above treatment.

The upper liquid phase 12 in the separation tank 1 may be recycled to the reaction system, if possible. If such recycling is impossible, the upper liquid phase 12 is supplied in another distillation apparatus 3 and separated into an azeotropic mixture of R-21 and HF, and HF containing substantially no R-21. The former is again cooled and recycled to the separation tank 1, while the latter HF is reused in the fluorination step. Thereby, substantially pure R-21 is recovered while utilizing whole HF effectively.

### Example 1

In a fluororesin vessel which had been evacuated, HF and R-21 were charged in a molar ratio of 70:30 and cooled at 0°C to cause phase separation. The molar ratio of HF to R-21 in the lower liquid phase was 4.2:95.8.

### Examples 2-4

In the same manner as in Example 1 but changing the cooling temperature from parts by weight to 10°C, 25°C or 70°C, the phase separation was carried out. The results are shown in the Table together with the result of Example 1.

**Table**

| Example No. | Phase separation temperature (°C) | Composition of lower liquid phase (HF/R-21) |
|---|---|---|
| 1 | 0 | 4.2/95.8 |
| 2 | 10 | 6.1/93.9 |
| 3 | 25 | 10.3/89.7 |
| 4 | 70 | 51.0/49.0 |

As understood from the results in the Table, the phase separation of the mixture considerably decreased the molar ratio of HF to R-21 in the lower liquid phase.

### Example 5

In a 1000 ml fluororesin vessel which had been evacuated, HF (140 g, 7.0 moles) and R-21 (309 g, 3.0 moles) were charged and cooled at 0°C. As the cooling proceeded, HF and R-21 were phase separated, and the whole volume of the lower liquid phase was recovered. The lower liquid phase contained 2.8 g (0.14 mole) of HF and 270.9 g (2.63 moles) of R-21. That is, the molar ratio of HF to R-21 was 5.05:94.9. This means the molar ratio of HF to R-21 greatly shifted to the R-21 side from the azeotropic composition.

The recovered lower liquid phase (200 g) was charged in a SUS distillation column. As the still temperature was gradually raised, an azeotropic mixture of HF and R-21 in a molar ratio of 70/30 was recovered from the condenser. When the distillation was withdrawn from the top of the column portion by portion, the top temperature became equal to the still temperature. Thereafter, from the still of the distillation column, about 190 g of R-21 containing about 10 ppm of HF was recovered.

## Claims

1. A process for removing hydrogen fluoride from a mixture comprising hydrogen fluoride and dichlorofluoromethane, which process comprises distilling said mixture and removing hydrogen fluoride in the form of an azeotropic mixture of hydrogen fluoride and dichlorofluoromethane.

2. A process according to Claim 1 for removing hydrogen fluoride from a mixture comprising hydrogen fluoride and dichlorofluoromethane, which process comprises cooling said mixture to a temperature of 70°C or lower to separate the mixture to form an upper liquid phase rich in hydrogen fluoride and a lower liquid phase rich in dichlorofluoromethane and distilling said lower liquid phase to remove hydrogen fluoride in the form of an azeotropic mixture of hydrogen fluoride and dichlorofluoromethane.

3. The process according to claim 2, wherein the cooling temperature is 50°C or lower.

4. The process according to claim 2, wherein the cooling temperature is from -10°C to 10°C.

5. The process according to claim 2, wherein said azeotropic mixture of hydrogen fluoride and dichlorofluoromethane is recycled to the cooling phase separation step.

6. An azeotropic mixture consisting essentially of dichlorofluoromethane and hydrogen fluoride.

## Patentansprüche

1. Verfahren zur Entfernung von Fluorwasserstoff aus einer Mischung, umfassend Fluorwasserstoff und Dichlorfluormethan, wobei das Verfahren das Destillieren der Mischung und Entfernen von Fluorwasserstoff in der Form einer azeotropen Mischung aus Fluorwasserstoff und Dichlorfluormethan umfaßt.

2. Verfahren nach Anspruch 1, zum Entfernen von Fluorwasserstoff aus einer Mischung, umfassend Fluorwasserstoff und Dichlorfluormethan, wobei das Verfahren das Kühlen der Mischung auf eine Temperatur von 70°C oder weniger, zum Trennen der Mischung, zur Bildung einer oberen flüssigen Phase, die reich ist an Fluorwasserstoff, und einer unteren flüssigen Phase, die reich ist an Dichlorfluormethan, und Destillieren der unteren flüssigen Phase, zur Entfernung von Fluorwasserstoff in der Form einer azoetropen Mischung aus Fluorwasserstoff und Dichlorfluormethan umfaßt.

3. Verfahren nach Anspruch 2, worin die Kühltemperatur 50°C oder weniger ist.

4. Verfahren nach Anspruch 2, worin die Kühltemperatur von -10°C bis 10°C ist.

5. Verfahren nach Anspruch 2, worin die azeotrope Mischung aus Fluorwasserstoff und Dichlorfluormethan zu dem Kühlphasentrennschritt zurückgeführt wird.

6. Azeotrope Mischung, im wesentlichen bestehend aus Dichlorfluormethan und Fluorwasserstoff.

## Revendications

1. Procédé de séparation de fluorure d'hydrogène d'un mélange comprenant du fluorure d'hydrogène et du dichlorofluorométhane, lequel procédé comprend la distillation dudit mélange et la séparation du fluorure d'hydrogène sous la forme d'un mélange azéotrope de fluorure d'hydrogène et de dichlorofluorométhane.

2. Procédé selon la revendication 1 de séparation de fluorure d'hydrogène d'un mélange comprenant du fluorure d'hydrogène et du dichlorofluorométhane, lequel procédé comprend le refroidissement dudit mélange à une température inférieure ou égale à 70°C pour séparer le mélange pour former une phase liquide supérieure riche en fluorure d'hydrogène et une phase liquide inférieure riche en dichlorofluorométhane, et la distillation de ladite phase liquide inférieure pour séparer le fluorure d'hydrogène sous la forme d'un mélange azéotrope de fluorure d'hydrogène et de dichlorofluorométhane.

3. Procédé selon la revendication 2, dans lequel la température de refroidissement est inférieure ou égale à 50°C.

4. Procédé selon la revendication 2, dans lequel la température de refroidissement est comprise entre -10°C et 10°C.

5. Procédé selon la revendication 2, dans lequel ledit mélange azéotrope de fluorure d'hydrogène et de dichlorofluorométhane est recyclé à l'étape de séparation de phases par refroidissement.

6. Mélange azéotrope consistant essentiellement en dichlorofluorométhane et en fluorure d'hydrogène.
